Europäisches Patentamt

European Patent Office

Office européen des brevets

(11) Veröffentlichungsnummer: **0 149 009**
**B1**

(12) **EUROPÄISCHE PATENTSCHRIFT**

(45) Veröffentlichungstag der Patentschrift:
02.11.88

(51) Int. Cl.⁴: **A 61 M 16/00**

(21) Anmeldenummer: 84111822.7

(22) Anmeldetag: 03.10.84

(54) Vorrichtung für die Zufuhr von Beatmungsgas in den geschlossenen Atemkreis eines medizinischen Beatmungsgerätes.

(30) Priorität: 17.01.84 DE 3401384

(43) Veröffentlichungstag der Anmeldung:
24.07.85 Patentblatt 85/30

(45) Bekanntmachung des Hinweises auf die Patenterteilung:
02.11.88 Patentblatt 88/44

(84) Benannte Vertragsstaaten:
DE FR GB IT NL SE

(56) Entgegenhaltungen:
DE-A-2 542 752
US-A-4 127 121
US-A-4 150 670

(73) Patentinhaber: Drägerwerk Aktiengesellschaft,
Moislinger Allee 53- 55, D-2400 Lübeck 1 (DE)

(72) Erfinder: **Wallroth, Carl Friedrich, Dr.,**
**Stresemannstrasse 1, D-2400 Lübeck (DE)**
Erfinder: **Frankenberger, Horst, Dr. rer.nat. Dipl.-**
**Phys., Promenadenweg 5, D-2407 Bad Schwartau**
**(DE)**
Erfinder: **Obermayer, Anton, Dipl.- Ing.,**
**Hindenburgstrasse 28b, D-8520 Erlangen (DE)**
Erfinder: **Waschmann, Michael, Dr.,**
**Rathenaustrasse 25, D-2400 Lübeck (DE)**
Erfinder: **Bayerlein, Jörg, Dr. Dipl.- Ing.,**
**Waldenburger Strasse 6, D-2406 Stockelsdorf (DE)**

## Beschreibung

Die Erfindung betrifft eine Vorrichtung für die Zufuhr von Beatmungsgas in den geschlossenen Atemkreis eines medizinischen Beatmungsgerätes, in dem das verbrauchte Beatmungsgas über einen Regelkreis dem mit einem Volumenantrieb versehenen Atemkreis zugeführt wird.

Eine derartige Vorrichtung für die Gaszufuhr in den geschlossenen Atemkreis eines medizinischen Beatmungsgerätes ist aus der US-A-4 127 121 bekannt.

Mit den vorgegebenen Vorrichtungen wird der Patient in einem geschlossenen Atemkreislauf beatmet. Die Zufuhr des notwendigen Sauerstoffs und Narkosegases erfolgt durch Dosiereinrichtungen. In diesen wird über Sensoren der Verbrauch festgestellt und die Differenz zum Sollwert nachdosiert. Der Verbrauch durch die Patienten ist von klinischer Bedeutung. Die Feststellung ist jedoch nur möglich, wenn das Beatmungssystem ohne Leckagen ist oder diese aber bekannt sind.

Bei der Vorrichtung nach der DE-A-2 542 752 kann nur im Strömungsfall und nur im Ausatemzyklus die mögliche unerwünschte Leckrate eines Ausatemventils bestimmt werden.

Aus der US-A-4 127 121 ist eine Vorrichtung für die überwachte Zufuhr von Sauerstoff und Narkosegas zu einem Patienten bekannt, die in Verbindung mit einem Beatmungsgerät benutzt wird. Das Beatmungsgerät enthält einen geschlossenen Atemkreislauf mit einem Patientenanschluß, einem Beatmungsbalg, einem Absorber für Kohlendioxid und je einer Dosiereinheit für die geregelte Zufuhr von Sauerstoff und von Narkosegas in den Atemkreislauf. Jede Dosiereinheit ist Endglied eines Regelkreises mit Sensor, Sollwertgeber, Komparator und Regler Der Sauerstoffregelkreis mißt mit einem in den Atemkreislauf eingesetzten Sauerstoffsensor die Sauerstoffkonzentration und hält diese durch angepaßte Zufuhr von Sauerstoff auf dem Sollwert. Der Narkosegasregelkreis mißt mit einem an dem Beatmungsbalg angeordneten Wegsensor dessen Scheitelhub und hält durch eine angepaßte Zufuhr von Narkosegas dessen Anteil auf dem Sollwert. Die Gaseinspeisungswerte der Regelkreise werden angezeigt und als Verbrauch des Patienten gedeutet.

Bei Leckagen im Atemkreislauf geben die angezeigten Werte nur die Summe aus dem Patientenverbrauch und den Leckverlusten an. Eine Aufschlüsselung ist nicht möglich. Eine Warnung vor der eingetretenen Leckage ist nicht gegeben.

Aufgabe der Erfindung ist, eine Vorrichtung für die Zufuhr von Beatmungsgas in den geschlossenem Atemkreis eines medizinischen Beatmungsgerätes der eingangs genannten Art so zu verbessern, daß es möglich ist, unter Ausnutzung der notwendigen Gasdosierung aus dem Gasverbrauch den Leckanteil sicher festzustellen.

Diese Aufgabe wird bei einer gattungsgemäßen Vorrichtung durch die kennzeichnenden Merkmale des Anspruchs 1 gelöst.

Die mit der Erfindung erzielten Vorteile bestehen insbesondere darin, daß die sichere Trennung der nachgeschobenen Gasmenge in den vom Patienten veratmeten Anteil und den Leckanteil möglich ist. Neben dem durch die dann mögliche sofortige Beseitigung von Leckagen erreichbaren geringsten Gasverbrauch werden vor allem Überbeanspruchungen des Patienten durch eine vielleicht zu geringe oder auch zu starke Gasversorgung verhindert.

Mit der Vorrichtung nach der Erfindung kann der Regelkreis entweder das Atemgas bereits gemischt aus seinen Komponenten Luft, Sauerstoff und Narkosegas für die Leckkontrolle benutzen oder mit getrennten Regelkreisen für die einzelnen Gase, wie z. B. Sauerstoff-Narkosegas, eines der jeweiligen Gase auf die gleiche Art für die Lecküberwachung benutzen.

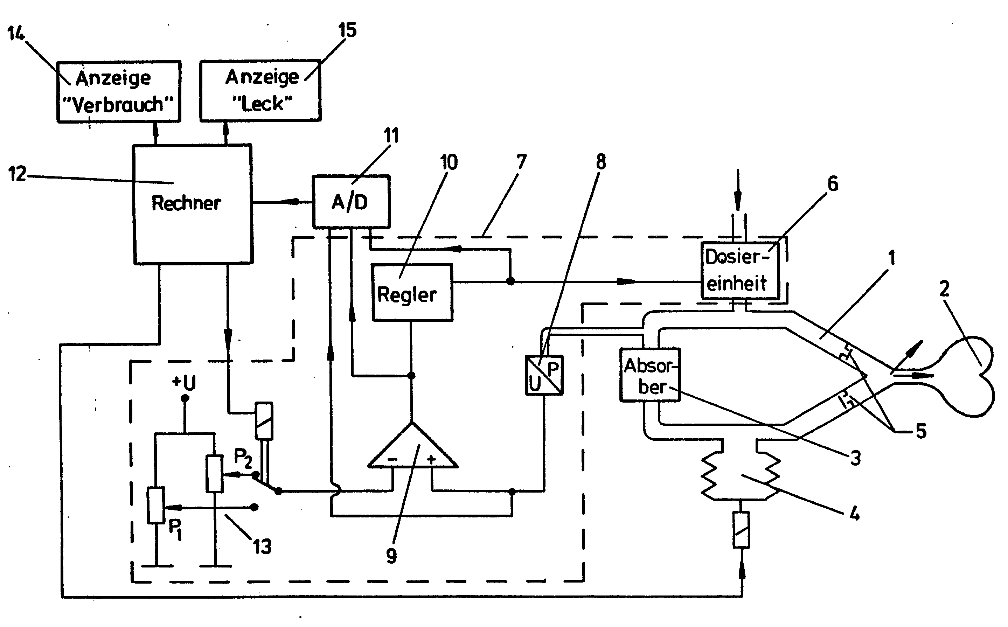

Ein Ausführungsbeispiel der Erfindung ist in der Zeichnung dargestellt und wird im folgenden beschrieben.

Die als Blockschema dargestellte Vorrichtung ist an den geschlossenen Atemkreis eines mechanischen Beatmungsgerätes angeschlossen. Der Atemkreis 1, geschlossen über die Lunge 2 des Patienten, enthält den $CO_2$-Absorber 3. Die Bewegung der Atemluft erfolgt in bekannter Weise mittels eines Beatmungsbalges als Volumenantrieb 4. Die Durchströmungsrichtung ist über Rückschlagventile 5 gesichert. Das verbrauchte Atemgas, der Sauerstoff und das Narkosegas, werden über eine Dosiereinheit 6 nachgeregelt, der es anteilmäßig zugeführt wird.

Der Regelkreis 7, dessen Endglied die Dosiereinheit 6 ist, wird über den Atemkreisdruck gesteuert. Er ist dazu über einen Druckaufnehmer 8 für das Istwertsignal mit dem Atemkreis 1 verbunden. Er enthält einen Differenzverstärker 9 mit einem nachgeschalteten Regler 10, der die Dosiereinheit 6 steuert.

Druckistwert (Ausgang Druckaufnehmer 8), Regelabweichung (Ausgang Differenzverstärker 9) und Dosierflow (Ausgang Regler 10) werden in einem Analog-Digital-Wandler 11 in ein digitales Signal umgewandelt und einem Rechner 12 zugeführt. Ein dem Differenzverstärker 9 vorgeschalteter Druckschalter 13 mit den einstellbaren Drucksollwerten $p_1$ und $p_2$ gibt diese Drucksollwerte für den Atemkreislauf vor, die dann über die Einspeisung E durch die Dosiereinheit 6 eingeregelt werden.

Der Rechner 12 gibt dazu die Signale zum Umschalten, wenn der Atemkreislauf seine geforderten Druckwerte erreicht hat, gleichzeitig wird der Volumenantrieb 4 stillgesetzt. Mit der erlaubten Voraussetzung, daß der Verbrauch V des Patienten in den hier gewählten Druckbereichen praktisch druckunabhängig ist, also bei beiden Drücken $p_1$ und $p_2$ gleich ist,

gelten mit dem Leck L im Atemkreis 1 folgende Gleichungen

$$E_1 = V + L (p_1 - p)$$
$$E_2 V + L (p_2 - p)$$

Daraus lassen sich das Leck L und der Verbrauch V des Patienten errechnen.

Sie werden im "Verbrauch" 14 und "Leck" 15 angezeigt.

## Patentansprüche

1. Vorrichtung für die Zufuhr von Beatmungsgas in den geschlossenen Atemkreis (1) eines medizinischen Beatmungsgerätes, in dem das verbrauchte Beatmungsgas über einen Regelkreis (7) dem mit einem Volumenantrieb (4) versenenen Atemkres zugeführt wird, dadurch gekennzeichnet, daß der Regelkreis (7) zur Aufnahme eines Istweertsignals mit einem Druckaufnehmer (8) sowie mit einem Differenzverstärker (9), einem Regler (10) und einer Dosiereinheit (6) und zur Festlegung von Sollwertsignalen mit einem auf mindestens zwei Vorgabewerte $p_1$ und $p_2$ umschaltbaren Druckschalter (13) an den Atemkreis (1) geschaltet ist, und daß ein Rechner (12) die über einen Analog-Digital-Wandler (11) empfangehen Daten des Regelkreises (7) derart verarbeitet, daß er den Volumenantrieb (4) jeweils bei Erreichen des von ihm über den Druckschalter (13) umgeschalteten Vorgabewertes $p_1$ oder $p_2$ stillsetzt und die von dem Regler (10) an die Dosiereinheit (6) abgegebenen, zur Aufrechterhaltung der Vorgabewerte erforderlichen, Eingabewerte ($E_1$, $E_2$) für die Atemgasdosierung zur Berechnung des Verbrauchs (V) und des Lecks (L) über das Gleichungspaar

$$E_1 = V + L (p_1 - p)$$
$$E_2 = V + L (p_2 - p)$$

heranzieht, wobei p de r Atmosphärendruck ist.

2. Vorrichtung für die Zufuhr von Beatmungsgas in der geschlossenen Atemkreis eines medizinichen Beatmungsgerätes nach Anspruch 1 dadurch gekennzeichnet, daß der Regelkreis (7) die Sauerstoffzufuhr regelt.

3. Vorrichtung für die Zufuhr von Beatmungsgas in den geschlossenen Atemkreis eines medizinischen Beatmungsgerätes nach Anspruch 1 dadurch gekennzeichnet, daß der Regelkreis (7) die Narkosegaszufuhr regelt.

## Claims

1. Device for the supply of respiratory gas into the closed respiratory circuit (1) of a medical breathing apparatus, in which the respiratory gas used is fed by way of a regulating circuit (7) to the respiratory circuit equipped with a volume actuator (4), characterised in that the regulating circuit (7) with a pressure pick-up (8) as well as with a differential amplifier (9), a regulator (10) and a dosing unit (6), for receiving an actual value signal and, for fixing desired value signals, with a pressure switch (13) which can be switched to at least two set values $p_1$ and $p_2$, is connected to the respiratory circuit (1), and that a computer (12) processes the data from the regulating circuit (7) received by way of an analog-digital-converter (11) in such way that it stops the volume actuator (4) in each case when reaching the set value $p_1$ or $p_2$ switched by it via the pressure switch (13) and draws up by way of the pair of equations

$$E_1 = V + L (p_1 - p)$$
$$E_2 = V + L (p_2 - p)$$

the input values ($E_1$, $E_2$), given by the regulator (10) to the dosing unit (6), and necessary for maintaining the set values, for the respiratory gas dosing for calculating the consumption (V) and the leakage (L), wherein p is the atmospheric pressure.

2. Device for the supply of respiratory gas into the closed respiratory circuit of a medical breathing apparatus according to claim 1, characterised in that the regulating circuit (7) regulates the supply of oxygen.

3. Device for the supply of respiratory gas into the closed respiratory circuit of a medical breathing apparatus according to claim 1, characterised in that the regulating circuit (7) regulates the supply of anaesthetic gas.

## Revendications

1. Dispositif pour l'apport de gaz respiratoire au circuit respiratoire fermé (1) d'un appareil respiratoire médical, dans lequel le gaz respiratoire consommé est amené par l'intermédiaire d'un circuit de régulation (7) au circuit respiratoire muni d'un entraînement volumique (4), caractérisé en ce que le circuit de régulation (7) est relié au circuit respiratoire (1) par un capteur de pression (8), un amplificateur différentiel (9), un régulateur (10) et une unité de dosage (6) pour recevoir un signal de valeur effective, ainsi que par un commutateur de pression (13) commutable à aux moins deux valeurs allouées $p_1$ et $p_2$ pour établir des signaux de valeur de consigne, et en ce qu'un calculateur (12) traite les données reçues du circuit de régulation (7) par l'intermédiaire d'un convertisseur analogique-numérique (11) de telle sorte qu'il arrête l'entraînement volumique (4) à l'atteinte respective de la valeur allouée $P_1$ ou $p_2$ commutée par lui par l'intermédiaire du commutateur de pression (13), et qu'il utilise, à l'aide de la paire d'équations ci-après, les valeurs d'entrée ($E_1$, $E_2$) - délivrées par le régulateur (10) à l'unité de dosage (6) et nécessaires pour maintenir les valeurs allouées - pour le dosage du gaz respiratoire, afin de calculer la consommation (V) et la fuite (L):

$$E_1 = V + L (p_2 - p)$$
$$E_2 = V + L (p_2 - p)$$

ou p est la pression atmosphérique.

2. Dispositif pour l'apport de gaz respiratoire au circuit respiratoire fermé d'un appareil respiratoire médical selon la revendication 1, caractérisé en ce que le circuit de régulation (7) régule l'apport d'oxygène.

3. Dispositif pour l'apport de gaz respiratoire au circuit respiratoire fermé d'un appareil respiratoire médical selon la revendication 1, caractérisé en ce que le circuit de régulation (7) régule l'apport de gaz anesthésique.

Anzeige "Verbrauch" — 14

Anzeige "Leck" — 15

12 — Rechner

11 — A/D

10 — Regler

9

P₂

P₁

13

+U

7

8

U P

6 — Dosier-einheit

Absor-ber

1

2

3

4

5

0149009